# EUROPÄISCHE PATENTSCHRIFT

(11) **EP 3 000 418 B1**
(45) Veröffentlichungstag und Bekanntmachung des Hinweises auf die Patenterteilung: **09.11.2016**
(21) Anmeldenummer: 14186613.7
(22) Anmeldetag: 26.09.2014
(51) Int. Cl.: A61B 17/22, A61B 17/225

(54) **Gerät zur Behandlung des menschlichen oder tierischen Körpers mit mechanischen Stößen**
Device for treatment of the human or animal body with mechanical impacts
Appareil à chocs mécaniques pour le traitement du corps humain ou animal

(43) Veröffentlichungstag der Anmeldung: 30.03.2016
(73) Patentinhaber: STORZ MEDICAL AG, 8274 Tägerwilen (CH)
(72) Erfinder: Novak, Pavel, 8234 Stetten (CH); Schulz, Johannes Manfred, 8274 Tägerwilen (CH); Swart, Stephan Gerhard, 47447 Moers (DE); Di Maio, Carlo, 47199 Duisburg (DE); Piontkowski, Ulrich, 74321 Bietigheim-Bissingen (DE)
(74) Vertreter: König Szynka Tilmann von Renesse Patentanwälte Partnerschaft mbB

(56) Entgegenhaltungen:
- DE-A1- 19 725 477
- US-A1- 2002 082 532
- US-A1- 2003 009 116
- US-A1- 2006 064 864

## Beschreibung

Die vorliegende Erfindung bezieht sich auf ein Gerät zur Behandlung des menschlichen oder tierischen Körpers durch Ausüben von Stößen auf die Körperoberfläche. Im Folgenden wird zur Vereinfachung vom Körper eines Patienten gesprochen, der bevorzugt menschlich ist.

Im Stand der Technik sind verschiedene Geräte des beschriebenen Grundtyps bekannt. Die DE 197 25 477 A1 beschreibt bspw. ein solches Gerät, bei dem durch die Kollision eines pneumatisch beschleunigten Schlagteils oder Projektils mit einem zunächst ruhenden Prallkörper oder Applikator eine Druckwelle ausgelöst wird, die dadurch in den Körper des Patienten eingekoppelt werden kann, dass eine Applikatorfrontfläche zum Zeitpunkt der Kollision auf den Patientenkörper aufgelegt wird. Dieser Gerätetyp leitet sich in seiner Entstehungsgeschichte von Lithotripsiegeräten ab, bei denen solche Druckwellen z. B. über eine lange stabförmige Sonde an der Frontfläche des Applikators auf einen Nierenstein oder Ähnliches übertragen werden können, um diesen zu desintegrieren.

Der Schwerpunkt bei der Darstellung liegt in jedem Fall auf der durch die Kollision erzeugten Druckwelle, die mehr oder weniger einer eigentlichen Stoßwelle ähneln soll, wie sie klassische Lithotripsiegeräte z. B. mit piezoelektrischen oder induktiven Aktuatoren und Fokussierung auf einen Stein erzeugen. Solche Druckwellen können Anstiegsflanken mit einer Breite im Bereich weniger µs und einer Amplitude im niedrigen zweistelligen MPa-Bereich haben (z. B. 2 µs und 15 MPa gemessen 1 cm vor der Frontfläche). In dem zitierten Dokument wird hingegen betont, dass die physikalisch an sich unvermeidliche makroskopische Schwerpunktbewegung des Applikators möglichst klein gehalten werden soll, weil sie als störend angesehen wird.

Als zweites Beispiel wird auf die DE 20 2004 011 323 U und, mit sehr ähnlichem Inhalt, die US 2011/0054367 A1 verwiesen. Dort wird ein hinsichtlich des technischen Aufbaus ähnliches Gerät beschrieben, bei dem allerdings u. a. die elastische Aufhängung des Applikators im Gehäuse auf größere Schwerpunktsbewegungen des Applikators ("Hübe") ausgelegt ist. Dort wird betont, dass die therapeutische Wirkung durchaus auch oder vorwiegend in den eigentlichen makroskopischen Stößen (also infolge des Hubes) gesehen werden kann, was auch von der Indikation abhängt.

Die vorliegende Erfindung richtet sich allgemein auf Geräte dieses Bautyps, und zwar sowohl hinsichtlich der Anwendung von Druckwellen als auch hinsichtlich der Anwendung "makroskopischer Stöße" des Applikator auf die Körperoberfläche.

Dabei liegt der Erfindung die Aufgabe zugrunde, ein solches Gerät hinsichtlich weitergehender Anwendungsmöglichkeiten weiterzubilden.

Die Aufgabe wird gelöst durch ein Gerät nach Anspruch 1. Erfindungsgemäß ist dabei der Applikator gebogen-flach und weist entsprechend der Biegung eine konkave und eine entgegengesetzt angeordnete konvexe Seitenfläche auf. Diese Seitenflächen entsprechen sich in der Form insoweit, dass der Applikator dazwischen eine begrenzte Stärke hat, also "flach" ist, was auf einen Grenzwert von höchstens 1/3 der Applikatorerstreckung entlang der konkaven und der konvexen Seitenfläche konkretisiert wird.

Mit dieser Vergleichsangabe ist die Länge (im geraden Sinn, nicht gekrümmt der Biegung nachfolgend) des Applikators vom Anfang bis zum Ende der beschriebenen gebogenen Form gemeint. Wenn diese also zum Beispiel, wie im Ausführungsbeispiel, etwa um die 30 mm beträgt, so soll die Stärke zwischen der konvexen und der konkaven Seitenfläche höchstens 10 mm betragen, vorzugsweise höchstens 1/4 oder sogar höchstens 1/5 davon, also im Beispielsfall höchstens 7,5 oder höchstens 6 mm. Dickere Bereiche des Applikators sind natürlich nicht grundsätzlich ausgeschlossen, etwa in der Umgebung seines Montageabschnitts zur Verbindung mit dem restlichen Gerät. Sie rechnen aber nicht zu der erfindungsgemäßen Form und vorzugsweise sind sie auch nicht distal, sondern allenfalls proximal von dieser vorgesehen (die Begriffe proximal und distal beziehen sich auf die Stoßrichtung).

Es hat sich herausgestellt, dass mit dieser flach gekrümmten Form die Arbeit des Therapeuten mit dem Gerät erleichtert wird. Insbesondere kann mit der gekrümmten Form hinter bestimmte Körperstrukturen "gegriffen" werden, ohne dass der Therapeut ein konventionelles Gerät dazu in unpraktischer Weise verkippt halten oder mit besonders hohem Druck auf die Körperoberfläche aufpressen müsste, um die Haut des Patienten ausreichend weit einzudrücken. Ein Beispiel ist der untere Rippenabschluss. Der Therapeut kann mit dem erfindungsgemäßen Applikator gewissermaßen um die untere Rippe herum greifen, wobei sich die konkave Seitenfläche an die Rippe anlegt, und behält dabei eine ergonomische und praktische Arbeitshaltung bei. Durch die begrenzte Stärke des Applikators muss dabei nur ein begrenzter Hautabschnitt eingedrückt und verschoben werden.

Ganz ähnlich bewährt sich die Erfindung bei der Behandlung von Muskeln innerhalb des Schulterblatts; auch hier kann der Therapeut mit dem Applikator um das Schulterblatt herumgreifen.

Außerdem eignet sich die konvexe Seite gleichzeitig als Schrägfläche für streichende Bewegungen auf der Körperoberfläche bei schräger Haltung des Geräts relativ zur Körperoberfläche. Dann greift sich das Gerät besser und kann in einer für den Therapeuten besser kontrollierbaren und bequemeren Weise über die Körperoberfläche geführt werden. Insbesondere können dabei Gewebepartien unter der Haut parallel massiert und/oder gewissermaßen vor dem Gerät hergeschoben werden, wobei sie gleichzeitig mit den Stößen und (in einem von Fall zu Fall unterschiedlichen Maß) Druckwellen beaufschlagt werden. Die konventionell bekannten Geräte mit mehr oder weniger flachen Frontflächen der Applikatoren sind dazu relativ schlecht geeignet, weil sie auf der Hautoberfläche nur bei mehr oder weniger senkrechter Stellung des Geräts relativ zur Körperoberfläche gehalten werden können.

Der Mechanismus zum Erzeugen der Stöße des Applikators ist in diesem Zusammenhang in unterschiedlichster Ausführung denkbar, insbesondere auch durch eine direkte Beaufschlagung des Applikators mittels eines elektromagnetischen Mechanismus oder auch eines Druckfluidpulses. Bevorzugt ist aber die aus den zitierten Dokumenten bereits bekannte Verwendung eines beschleunigten Projektils zur Kollision mit dem Applikator, womit relativ heftige und (im Sinn der Applikatorgeschwindigkeit) schnelle Stöße realisiert werden können und auch die gleichzeitige oder sogar schwerpunktmäßige Verwendung von Druckwellen möglich ist. Das Projektil wiederum lässt sich ebenfalls in unterschiedlicher Weise beschleunigen, insbesondere auch elektromagnetisch, wobei auch hier eine pneumatische Beaufschlagung des Projektils gemäß den zitierten Schriften bevorzugt ist.

Der Begriff "Applikator" bezeichnet hier übrigens nicht zwingend ein einstückiges Teil. Es ist denkbar und anwendungsabhängig auch bevorzugt, dass das Projektil z. B. auf ein erstes Teil schlägt, das den Stoß und/oder die Druckwelle auf ein zweites Applikatorteil überträgt, das seinerseits mit der Haut des Patienten in Kontakt steht. Im Stand der Technik ist auch gelegentlich von einem Zwischenstück zwischen Applikator und Projektil die Rede; hier wird dann von einem mehrteiligen Applikator gesprochen, wobei die beiden Applikatorteile fest verbunden sein können, aber nicht sein müssen.

Wie eingangs bereits erwähnt, kann ein relativ großer Hub des Applikators durchaus gewünscht sein, wobei im vorliegenden Zusammenhang das Gerät vorzugsweise so ausgelegt ist, dass Hübe über 1 mm erreichbar sind, wie schon in der US 2011/0054367 A1 ausgeführt.

Vorzugsweise weist der erfindungsgemäße Applikator genau den einen beschriebenen Krümmungsverlauf auf, also keinen weiteren mit anderem Krümmungssinn, etwa entgegengesetzt oder in einer deutlich abweichenden Ebene der Krümmung. Eine solche einfache Applikatorform ist nicht nur vorteilhaft hinsichtlich der Herstellbarkeit, sondern auch praktisch und komplikationsfrei in der Anwendung.

Ferner liegen die konvexe und die konkave Seitenfläche vorzugsweise so, dass eine Schnittebene, in der sich die jeweilige Krümmung besonders ausgeprägt zeigt, die Stoßrichtung enthält und die auftretenden Krümmungsmittelpunkte in Bezug auf die Stoßrichtung seitlich neben dem Applikator liegen. In anderen Worten krümmt sich die Applikatorform so, dass die konvexe Seitenfläche in Bezug auf die Stoßrichtung zu einer Seite (und nicht in Bezug auf die Stoßrichtung nach vorne oder nach hinten) und die konkave Seitenfläche zu der entgegengesetzten Seite zeigt. Wenn man sich die Applikatorform in einer Schnittebene vereinfacht wie eine Banane vorstellt (vgl. Ausführungsbeispiel), um hier nur ein Beispiel zu nennen, dann liegt ein Ende dieser Banane proximal und das andere distal zum Gerät und liegt nicht etwa zum Beispiel die Mitte der Banane proximal zum Gerät und weisen die beiden Enden davon weg.

Ferner ist die Krümmung vorzugsweise im proximalen Bereich des Applikators stärker ausgeprägt als im distalen, vergrößern sich also die jeweiligen Krümmungsradien mit zunehmendem Abstand von dem Gerät, wozu ebenfalls auf das Ausführungsbeispiel verwiesen werden kann.

Eine weitere bevorzugte Charakterisierung betrifft eine bisher noch nicht angesprochene Dimension, nämlich die Breite senkrecht zur Stoßrichtung und zu der Ebene, in der sich die Krümmung zeigt. Hier ist der Applikator mindestens so breit wie die angesprochene (dem Begriff "flach" zugrunde liegende) Stärke, vorzugsweise mindestens 1 1/2 mal so breit, oder sogar mindestens 2 mal, 2 1/2 mal oder sogar 3 mal so breit. Bevorzugt sind also "flach-breite" und nicht etwa stabförmige Geometrien (weswegen sich der obige Vergleich mit der Banane auf einen Schnitt bezieht).

Schließlich ist der Applikator vor allem in seinem vorderen Bereich, konkret in der distalen Hälfte (in Bezug auf die Längserstreckung in Stoßrichtung) "kantenfrei" im Sinne von Mindestkrümmungsradien von 1,5 mm in zumindest einer Schnittebene, vorzugsweise der, in der sich die beschriebene Krümmung zeigt, und besonders bevorzugter Weise noch in einer zweiten Schnittebene senkrecht dazu, vgl. Ausführungsbeispiel.

Insgesamt ergeben sich für den Applikator hinsichtlich seines von dem übrigen Gerät freien (insbesondere daraus hervorstehenden) Teils folgende bevorzugte Dimensionen:

Die Länge ist vorzugsweise größer als 10 mm, 20 mm, 30 mm oder sogar 35 mm und vorzugsweise kleiner als 100 mm, 90 mm, 80 mm, 70 mm, 65 mm oder sogar 60 mm. Beim Ausführungsbeispiel beträgt sie etwa 50 mm. Dabei beträgt die Länge des eigentlichen flach-gekrümmten Abschnitts beim Ausführungsbeispiel etwa 32 mm (in Stoßrichtung) und allgemein vorzugsweise mehr als 5 mm, 10 mm, 15 mm oder sogar 20 mm und vorzugsweise weniger als 60 mm, 50 mm, oder sogar 40 mm.

Die Breite liegt vorzugsweise über 5 mm, 10 mm oder sogar 15 mm und andererseits vorzugsweise unter 80 mm, 60 mm, 40 mm oder sogar 30 mm und beträgt beim Ausführungsbeispiel 20 mm.

Die Stärke beträgt vorzugsweise mindestens 2 mm, 3 mm oder sogar 3,5 mm und vorzugsweise weniger als 10 mm, 9 mm, 8 mm oder sogar 7 mm und beim Ausführungsbeispiel 5 mm.

Ferner sollte der vorderste Teil (distale Teil) in Bezug auf die Bewegungsrichtung einen Winkel von mindestens 20° oder sogar 25° und höchstens 80°, 70° oder sogar 60° haben. Beim Ausführungsbeispiel beträgt er im letzten Abschnitt etwa 40°.

Vorzugsweise wird dieser Winkel über eine Krümmung erreicht, die sich ebenfalls vorzugsweise vom proximalen Abschnitt bis zum distalen Abschnitt verflacht (der Krümmungsradius nimmt also zu). Im distalen Bereich kann ein bevorzugter Krümmungsradius typischerweise über 15 mm, 20 mm oder sogar 25 mm und unter 80 mm, 75 mm, 70 mm, 65 mm, 60 mm oder sogar 55 mm liegen; beim Ausführungsbeispiel beträgt der Krümmungsradius dort 40 mm. Andererseits liegt er beim Ausführungsbeispiel im proximalen Abschnitt bei nur 5 mm und vergrößert sich von dort ausgehend.

Schließlich liegt das distale Applikatorende im Bezug auf die Stoßrichtung vorzugsweise ungefähr mittig, und zwar vorzugweise innerhalb von 15 mm, oder sogar nur 5 mm um eine Mittellängsachse des Gerätes herum.

Vorbekannte Applikatoren bestanden im Regelfall aus rostfreiem Stahl. Im vorliegenden Fall kommen grundsätzlich auch Metalle, so auch rostfreier Stahl, in Betracht. Daneben sind als metallische Materialien Aluminium und Titan zu nennen, wobei beide eine relativ geringe Massendichte haben und damit relativ leichte Applikatoren ermöglichen. Das kann den Vorteil haben, bei gegebener Geometrie eine stärkere Beschleunigung und damit auch größere Hubauslenkung des Applikators zu ermöglichen, was im vorliegenden Fall gewünscht sein kann. Titan zeichnet sich zudem durch eine besonders hohe mechanische Belastbarkeit aus, eignet sich also besonders für Anwendungen mit vergleichsweise hohen Projektilgeschwindigkeiten und/oder Projektilmassen, aber nicht nur hierfür. Oft ist auch die geringe Dichte und/oder gute physiologische Verträglichkeit ausschlaggebend . In Betracht kommen ferner Keramiken, vgl. Anmeldenummer 08 003 840.9 / EP 2 095 843, und Kunststoffe. Im Vergleich zu rostfreiem Stahl weisen auch diese den Vorteil geringer Massendichte auf. Sie haben ferner eine geringere Wärmeleitfähigkeit als Metalle, so dass der Patient den Applikator subjektiv als wärmer empfindet. Das gilt vor allem für Kunststoffe. Kunststoffe kommen insbesondere auch dann in Betracht, wenn auf die Einkopplung einer Druckwelle geringerer Wert gelegt wird, weil, jedenfalls bei einer größeren Stärke des Kunststoffs, im Vergleich zu den vorgenannten Materialien etwas größere Wellenleitungsverluste zu erwarten sind.

Im Folgenden wird die Erfindung auch anhand eines Ausführungsbeispiels näher erläutert, dessen einzelne Merkmale im Rahmen des geltenden Anspruchs 1 auch unabhängig voneinander und in anderen Kombinationen erfindungswesentlich sein können.
- Figur 1: zeigt ein Druckwellengerät als erstes Ausführungsbeispiel der Erfindung mit einem in Fig. 2 näher dargestellten Applikator.
- Figur 2: zeigt den Vorderteil des Applikators des Geräts aus Fig. 1 in gleicher Blickrichtung wie in Fig. 1 (aber aufrecht) und in einer Längsschnittdarstellung in Teildarstellung a, in einer Draufsicht mit um 90° um die Längsachse verdrehter Blickrichtung in Teildarstellung b, in einer weiteren Darstellung c in Längsrichtung des Geräts gesehen und schließlich in Teildarstellung d in perspektivischer Ansicht.

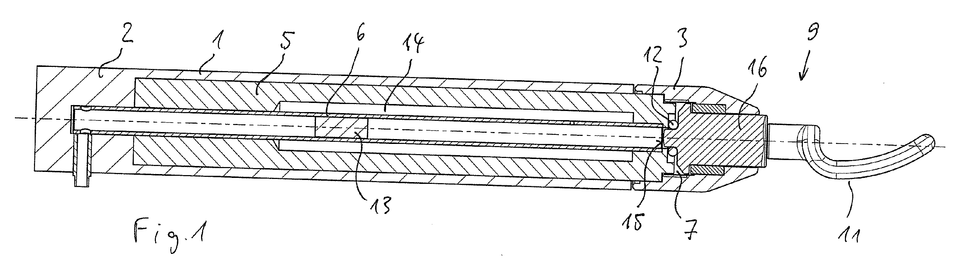

Figur 1 zeigt ein erstes Ausführungsbeispiel der Erfindung. Es handelt sich um ein Gerät zur Einkopplung von Stößen und unfokussierten (so genannten radialen) mechanischen Druckwellen in den menschlichen oder tierischen Körper.

Ein Rohrstück 1 bildet gemeinsam mit einer in der Anwendung körperabgewandten und mit dem Rohrstück 1 integrierten Zuluftkappe 2 und einer in der Anwendung körperzugewandten Applikatorkappe 3 ein Gehäuse. Die Zuluftkappe 2 enthält einen Druckluftanschluss 4 für eine pneumatische Versorgung. In an sich bekannter Weise ist an diesen Druckluftanschluss 4 über eine pneumatische Versorgungsleitung ein von einer Ansteuereinheit gesteuertes Ventil, insbesondere Magnetventil, angeschlossen, das in einem gleich bleibenden iterativen Takt zwischen etwa 1 Hz und 50 Hz Druckluftpulse über den Druckluftanschluss einkoppelt. Das Ventil ist nicht gezeigt und kann auch in dem dargestellten Gerät selbst eingebaut sein.

Das Gerät ist im Übrigen als mit der Hand einer Bedienungsperson zu haltendes Gerät ausgebildet, das über die erwähnte Pneumatikleitung an eine nicht gezeigte Basisstation mit der Ansteuereinheit und dem Kompressor angeschlossen ist und auf den Patienten manuell aufgesetzt werden kann. Es eignet sich besonders für die Behandlung von Körperpartien hinter körpereigenen Hindernissen wie Rippen oder Schulterblättern.

In dem Gehäuse ist über einen Einsatz 5 ein Führungsrohr 6 gehalten, dessen bei der Anwendung körperfernes Ende in der Zuluftkappe 2 endet und dort mit dem Druckluftanschluss 4 kommuniziert. Das in der Anwendung körperseitige Ende des Führungsrohres 6 endet in einem Teil des Einsatzes 5, der in die Applikatorkappe 3 hineinragt, und zwar kurz vor dem dortigen Ende des Einsatzes 5 und einem Innenraum 7 in der Applikatorkappe 3.

In dem Innenraum 7, der in eine in der Anwendung körperseitige Applikatoröffnung übergeht, ist ein erster und in Fig. 1 schraffierter Teil eines Applikator 9 aufgenommen. Dieser stützt sich über ein elastisches Schlauchelement 10 aus einem Elastomer an einer radialen Schulter ab. Ein zur körperfernen Seite gerichtetes und die Aufprallfläche beinhaltendes Ende 15 des Applikators 9 stützt sich über einen O-Ring 12 an dem Einsatz 5 ab, und zwar an einer das bereits erwähnte Ende des Einsatzes 5 umgebenden Stirnfläche. Dabei liegt der O-Ring 12 zwischen dieser Stirnfläche und einer Schulter des Applikators 9. Die Applikatoröffnung 8 dient dabei zu einer in der Längsrichtung verschiebbaren Führung des Applikators 9 und fixiert diesen quer zur Längsrichtung. Die Axialverschiebbarkeit ist nur durch die Nachgiebigkeit des Elastomerelements 10 begrenzt und kann bei einem in Luft betriebenen Gerät relativ zum Restgerät auch deutlich über 1 mm liegen.

Der Applikator 9 weist als zweiten Teil das nicht schraffierte Element 11 auf, das den eigentlichen auf die Haut aufzusetzenden Applikator bildet. Der Applikator 9 ist durch Abschrauben der Applikatorkappe 3 austauschbar.

In dem angrenzenden Bereich des Führungsrohres 6 ist ein in Figur 1 mit dem Applikator 9 in Kontakt stehendes Projektil 13 eingesetzt. Dieses passt (in Bezug auf das Führungsrohr und die im Wesentlichen zylindrische Geometrie des Projektils 13) radial mit geringem Spiel hinein. Das Projektil 13 kann durch Druckunterschiede der Luftsäule in dem Führungsrohr 6 vor und hinter ihm (d. h. in Figur 1 rechts und links des Projektils 13) in dem Führungsrohr hin- und herbewegt werden und insbesondere auf den Applikator 9 zu beschleunigt werden. Hierzu wird es aus einer Ausgangsposition (nicht gezeigt) in Figur 1 links durch einen Druckluftstoß durch den Druckluftanschluss 4 beschleunigt und trifft mit seiner dem Applikator 9 zugewandten Frontfläche auf den Applikator 9 auf, und zwar auf eine körperabgewandte Prallfläche 15 davon.

Die Rückbewegung des Projektils 13 erfolgt zusätzlich zu einem Zurückprallen nach der Kollision durch ein Rückströmen der Luft aus einer das Führungsrohr 6 innerhalb des Einsatzes 5 umgebenden Staukammer 14. In diese wird die Luft bei der Beschleunigung des Projektils 13 in Richtung zu dem Prallkörper 9 verdrängt und damit dort komprimiert. Wenn das Magnetventil den Druck wegschaltet und gleichzeitig den Raum hinter dem Projektil entlüftet, wird das Projektil 13 damit in die Ausgangsstellung zurückbewegt. Dies kann natürlich auch durch eine zusätzliche oder alternative Druckbeaufschlagung der Staukammer 14 oder eines anderen Luftvolumens körperseitig von dem Projektil 13 erfolgen. Das in der Anwendung körperferne Ende des Führungsrohres 6 endet in einem Magnethalter für das Projektil 13.

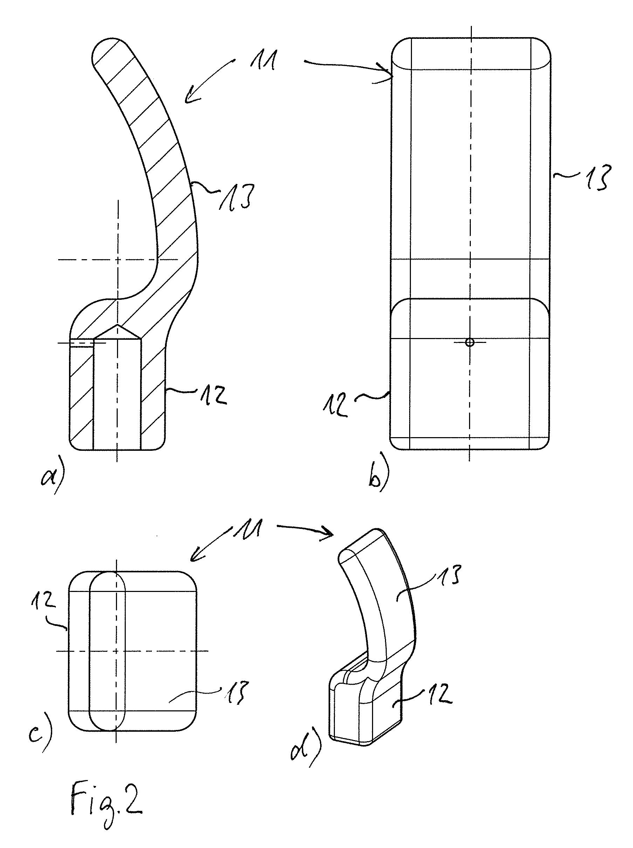

Wenn man sich den schraffierten Teil 16 des Applikators 9 in Fig. 1 mit einer zum Beispiel leicht konvexen Begrenzungslinie abgeschlossen vorstellt, würde er eine konventionelle Applikatorform bilden. In dieser Form würde sich der Applikator 9 vor allem für das vertikale Aufsetzen auf die Körperoberfläche des Patienten eignen. Erfindungsgemäß ist der Applikator 9 nun in Richtung zu dem Patientenkörper ergänzt, und zwar um den nicht schraffierten Teil 11. Dieser hat in einer Richtung senkrecht zur Längsrichtung gesehen die in Figur 1 angegebene Form und in der zweiten Richtung senkrecht zur ersten und zur Längsrichtung die in Figur 2b dargestellte Form. Dabei ist der Applikator 9 in Figur 1 ungefähr im richtigen Größenverhältnis zum Restgerät dargestellt, in Figur 2 aber vergrößert.

In Figur 2a sieht man einen Längsschnitt durch den vorderen Applikatorteil 11. Dabei ist an der gerätezugewandten (proximalen) Seite eine Hohlaufnahme eingezeichnet, die zur Verbindung mit dem restlichen Applikator dienen kann, hier aber keine wesentliche Rolle spielt. Jedenfalls schließt sich an einen im Querschnitt senkrecht zur Stoßrichtung rechteckig (mit abgerundeten Ecken) Sockel 12 eine gegenüber dem Sockel zunächst seitlich versetzte und flach-gekrümmt verlaufende Form 13 an.

Die flach gekrümmte Form 13 startet von dem Sockel ausgehend mit einem inneren Krümmungsradius von 5 mm, der sich bis zu der Spitze der flach-gekrümmten Form 13 allmählich vergrößert bis auf 40 mm, wobei die Spitze selbst mit einem Radius von 2,5 mm verrundet ist. Diese Spitze ist gegenüber der Stoßrichtung um 40° verkippt. Der Krümmungsradius an der Außenseite der flach-gekrümmten Form 13 beträgt etwa 45 mm und die in Fig. 2a im Schnitt erkennbare Materialstärke 5 mm. Die in Fig. 2b sichtbare Breite beträgt hingegen 20 mm über den gesamten Bereich der flach-gekrümmten Form 13, wobei nur die Ecken abgerundet sind. Wenn man auf den oberen Teil des Applikators 11 aus Fig. 2b vertikal schaut, also gegenüber der Blickrichtung in Fig. 2b um 40° verdreht, entspricht dieser Verrundungsradius in den oberen beiden Ecken 5 mm. Fig. 2c zeigt eine Draufsicht, also eine Ansicht in Stoßrichtung, und Fig. 2d eine perspektivische Ansicht, wobei die konkave Seite nach hinten links und die konvexe nach vorne rechts weist. Man erkennt, dass die Spitze in einem Bereich um etwa beidseits 5 mm um die Längsachse (in Stoßrichtung) liegt.

Die Figuren 1, 2a und 2d, zeigen, dass der vordere Teil 11 des Applikators 9 genau einen Krümmungsverlauf mit einer konkaven Seitenfläche (in Figur 2a nach links) und einer konvexen (nach rechts) aufweist, wobei die Figur 2a außerdem zeigt, dass in der dortigen Schnittebene die Krümmungsmittelpunkte der Konkavität und Konvexität seitlich neben dem Applikator 9 bzw. 11 liegen, nämlich links daneben. Ferner erkennt man, dass die Krümmung von einem proximalen zu einem distalen Bereich abnimmt.

Mit einem solchen Vorderteil 11 des Applikators kann die behandelnde Person um bestimmte Körperstrukturen wie z. B. Rippen und Schulterblätter herumgreifen und ist damit für bestimmte Anwendungsfälle besonders praktisch ausgestattet.

## Patentansprüche

1. Gerät zur Behandlung des menschlichen oder tierischen Körpers mit
- einem Applikator (9,11,16) zum Auflegen auf den Körper von außen,
- einem Gehäuse (1,2,3), in dem der Applikator (9,11,16) gehalten ist, und - einem Mechanismus (6,13) zum Erzeugen von Stößen des Applikators (9,11,16) relativ zu dem Gehäuse (1,2,3) in einer Stoßrichtung, so dass die Stöße bei dem Auflegen in den Körper eingekoppelt werden können,
**dadurch gekennzeichnet, dass** der Applikator (9,11,16) eine gebogen-flache Form aufweist mit
- einer konkaven Seitenfläche nach einer Seite,
- einer konvexen Seitenfläche nach einer dazu entgegengesetzten Seite
- und einer Stärke zwischen diesen Seitenflächen, die höchstens ein Drittel der Erstreckung des Applikators entlang der konkaven und der konvexen Seitenfläche beträgt.

2. Gerät nach Anspruch 1, bei dem der Mechanismus (6,13) zum Erzeugen der Stöße ein Projektil (13) und eine Einrichtung (6) zum Beschleunigen des Projektils (13) in solcher Weise aufweist, dass das Projektil (13) auf den Applikator (9,11,16) schlägt und dadurch den Stoß erzeugt, vorzugsweise eine pneumatische Einrichtung zur Beschleunigung des Projektils (13).

3. Gerät nach Anspruch 1 oder 2 mit genau einem durch die konkave und die konvexe Seitenfläche bedingten Krümmungsverlauf.

4. Gerät nach einem der vorstehenden Ansprüche, bei dem sich die Konvexität und die Konkavität der Seitenflächen in einer Schnittebene zeigen, die die Stoßrichtung enthält und sich die Krümmungsmittelpunkte der Konkavität und der Konvexität im Bezug auf die Stoßrichtung seitlich neben dem Applikator befinden.

5. Gerät nach einem der vorstehenden Ansprüche, bei dem die Konkavität und die Konvexität in einem bezüglich der Stoßrichtung proximalen Bereich kleinere Krümmungsradien aufweisen als in einem diesbezüglich distalen Bereich.

6. Gerät nach einem der vorstehenden Ansprüche, bei dem die Breite des Applikators (9,11,16) senkrecht zu der Stoßrichtung und senkrecht zu einer Schnittebene, die den durch die konkave und konvexe Seitenfläche bedingten Krümmungsverlauf zeigt, mindestens so groß ist wie die Stärke des Applikators (9,11,16) zwischen diesen Seitenflächen.

7. Gerät nach einem der vorstehenden Ansprüche, bei dem der Applikator (9,11,16) in seiner distalen Hälfte in zumindest einer Schnittebene nur Krümmungsradien von mindestens 1,5 mm aufweist, vorzugsweise in zwei Schnittebenen.

8. Gerät nach einem der vorstehenden Ansprüche, bei dem der Applikator (9,11,16) mit seinem von anderen Teilen des Gerätes freien Bereich (11) eine Gesamtlänge in der Stoßrichtung zwischen 10 mm und 100 mm aufweist.

9. Gerät nach einem der vorstehenden Ansprüche, bei dem der Applikator (9,11,16) mit seinem von anderen Teilen des Gerätes freien Bereich (11) eine Stärke zwischen der konkaven und der konvexen Seitenfläche zwischen 2 mm und 10 mm aufweist.

10. Gerät nach einem der vorstehenden Ansprüche, bei dem der Applikator (9,11,16) mit seinem von anderen Teilen des Gerätes freien Bereich (11) eine Gesamtbreite senkrecht zur Stoßrichtung und senkrecht zu einer Schnittebene, die den durch die konkave und konvexe Seitenfläche bedingten Krümmungsverlauf zeigt, zwischen 5 mm und 80 mm aufweist.

11. Gerät nach einem der vorstehenden Ansprüche, bei dem der Applikator (9,11,16) in seinem zum Auflegen ausgelegten Teil (11) besteht aus Metall, insbesondere Aluminium oder Titan, Kunststoff oder Keramik.

12. Gerät nach einem der vorstehenden Ansprüche, das ausgelegt ist für einen Hub des Stoßes in der Stoßrichtung relativ zu dem Gehäuse von mindestens 1 mm.

## Claims

1. An apparatus for treating a human or animal body, having
- an applicator (9,11,16) for being placed on said body from the outside,
- a housing (1,2,3) in which said applicator (9,11,16) is held, and
- an apparatus (6,13) for generating strokes of said applicator (9,11,16) with respect to said housing (1,2,3) in a stroke direction so that said strokes can be coupled into said body when said applicator is placed on said body,
**characterized in that** said applicator (9,11,16) has a curved-flat shape with
- a concave lateral face to one side,
- a convex lateral face to a side opposite thereto
- and a size between said lateral faces, which amounts to one third at maximum of an extension of said applicator along said concave and said convex lateral face.

2. The apparatus according to claim 1, wherein said apparatus (6,13) for generating said strokes comprises a projectile (13) and a device (6) for accelerating said projectile (13) in such a way that said projectile (13) hits said applicator (9,11,16) and generates said stroke, thus, preferably a pneumatic device for accelerating said projectile (13).

3. The apparatus according to claim 1 or 2, having exactly one gradient of curvature being a result of said concave and said convex lateral face.

4. The apparatus according to one of the preceding claims, wherein the convexity and the concavity of said lateral faces shows in a sectional plane containing said stroke direction, wherein centers of curvature of said concavity and said convexity are respectively located aside said applicator laterally with respect to said stroke direction.

5. The apparatus according to one of the preceding claims, wherein the concavity and the convexity have smaller radii of curvature in a region being proximal with respect to said stroke direction than in a region being distal with respect thereto.

6. The apparatus according to one of the preceding claims, wherein a width of said applicator (9,11,16) perpendicularly to said stroke direction and perpendicularly to a sectional plane showing the gradient of curvature which is a result of said concave and said convex lateral face is at least as large as said size of said applicator (9,11,16) between said lateral faces.

7. The apparatus according to one of the preceding claims, wherein radii of curvature amount to 1.5 mm at minimum in a distal half of said applicator in at least one sectional plane, preferably in two sectional planes.

8. The apparatus according to one of the preceding claims, wherein a region (11) of said applicator (9,11,16), which is free of other parts of said apparatus, has a total length in said stroke direction between 10 mm and 100 mm.

9. The apparatus according to one of the preceding claims, wherein a region (11) of said applicator (9,11,16), which is free of other parts of said apparatus, has a size between said concave and said convex lateral face of between 2 mm and 10 mm.

10. The apparatus according to one of the preceding claims, wherein a region (11) of said applicator (9,11,16), which is free of other parts of said apparatus, has a total width of between 5 mm and 80 mm perpendicularly to said stroke direction and perpendicularly to a sectional plane showing the gradient of curvature which is a result of said concave and said convex lateral face.

11. The apparatus according to one of the preceding claims, wherein a part (11) of said applicator (9,11,16), which is adapted for being placed on said body, is made of metal, in particular aluminium or titanium, synthetic material or ceramics.

12. The apparatus according to one of the preceding claims, which is adapted for a travel of said stroke of 1 mm at minimum in said stroke direction relatively to said housing.

## Revendications

1. Appareil pour le traitement du corps humain ou animal, comportant:
- un applicateur (9, 11, 16) destiné à une application sur ledit corps depuis l'extérieur,
- un boîtier (1, 2, 3) destiné à retenir l'applicateur (9, 11, 16), et
- un mécanisme (6, 13) permettant à l'applicateur (9, 11, 16) d'exercer des coups par rapport au boîtier (1, 2, 3) dans une direction d'impact, de manière que les coups peuvent être répercutés dans le corps suite à ladite application,
**caractérisé en ce que** l'applicateur (9, 11, 16) présente une forme courbe-plate comportant:
- une surface latérale concave sur un côté,
- une surface latérale convexe sur un côté opposé,
- et une épaisseur entre ces surfaces latérales correspondant au maximum au tiers de l'étendue de l'applicateur le long des surfaces latérales concave et convexe.

2. Appareil selon la revendication 1, dans lequel le mécanisme (6, 13) permettant d'exercer des coups présente un projectile (13) et un dispositif (6) permettant une accélération du projectile (13) de telle manière que le projectile (13) frappe l'applicateur (9, 11, 16) en produisant ainsi ledit coup, s'agissant de préférence d'un dispositif pneumatique d'accélération du projectile (13).

3. Appareil selon la revendication 1 ou 2, comportant exactement une courbure résultant de la surface latérale concave et de la surface latérale convexe.

4. Appareil selon l'une quelconque des revendications précédentes, dans lequel la convexité et la concavité des surfaces latérales se manifestent dans un plan de coupe contenant la direction d'impact et les centres de courbure de la concavité et de la convexité se trouvent, eu égard à la direction d'impact, latéralement à côté de l'applicateur.

5. Appareil selon l'une quelconque des revendications précédentes, dans lequel la concavité et la convexité présentent de plus petits rayons de courbure dans une zone dite proximale que dans une zone dite distale eu égard à la direction d'impact.

6. Appareil selon l'une quelconque des revendications précédentes, dans lequel la largeur de l'applicateur (9, 11, 16) perpendiculairement à la direction d'impact et perpendiculairement à un plan de coupe manifestant la courbure résultant des surfaces latérales concave et convexe, est au moins aussi grande que l'épaisseur de l'applicateur (9, 11, 16) entre ces surfaces latérales.

7. Appareil selon l'une quelconque des revendications précédentes, dans lequel l'applicateur (9, 11, 16), dans sa moitié distale, dans au moins un plan de coupe et de préférence dans deux plans de coupe, ne présente que des rayons de courbure d'au moins 1,5 mm.

8. Appareil selon l'une quelconque des revendications précédentes, dans lequel l'applicateur (9, 11, 16), par sa zone (11) libre de tout autre composant de l'appareil, présente une longueur totale, dans la direction d'impact, faisant entre 10 mm et 100 mm.

9. Appareil selon l'une quelconque des revendications précédentes, dans lequel l'applicateur (9, 11, 16), par sa zone (11) libre de tout autre composant de l'appareil, présente une épaisseur, entre la surface latérale concave et la surface latérale convexe, faisant entre 2 mm et 10 mm.

10. Appareil selon l'une quelconque des revendications précédentes, dans lequel l'applicateur (9, 11, 16), par sa zone (11) libre de tout autre composant de l'appareil, présente une largeur totale, perpendiculairement à la direction d'impact et perpendiculairement à un plan de coupe manifestant la courbure résultant des surfaces latérales concave et convexe, faisant entre 5 mm et 80 mm.

11. Appareil selon l'une quelconque des revendications précédentes, dans lequel l'applicateur (9, 11, 16), dans sa partie (11) conçue pour l'application sur le corps, se compose de métal, notamment d'aluminium ou de titane, de plastique ou de céramique.

12. Appareil selon l'une quelconque des revendications précédentes, conçu pour une course d'impact, dans la direction d'impact par rapport au boîtier, qui est d'au moins 1 mm.
